# EUROPEAN PATENT APPLICATION

(11) **EP 3 178 394 A1**
(43) Date of publication of application: **14.06.2017**
(21) Application number: 14899546.7
(22) Date of filing: 25.09.2014
(51) Int. Cl.: A61B 5/055

(54) **NUCLEAR MAGNETIC RESONANCE IMAGING SYSTEM FOR INFANTS AND METHOD IMAGING THEREOF**

(30) Priority: 06.08.2014 CN 201410384046
(71) Applicant: Jiangsu Magspin Instrument Co., Ltd., Kunshan, Jiangsu 215300 (CN)
(72) Inventor: ZHAO, Huawei, Kunshan Jiangsu 215300 (CN); WANG, Hua, Kunshan Jiangsu 215300 (CN)
(74) Representative: Lavoix
(86) International application number: PCT/CN2014/087368
(87) International publication number: WO 2016/019619

(57) **Abstract**

A magnetic resonance imaging (MRI) system for infants and children and imaging method thereof are disclosed. The system includes: a base (10); a housing (20), with a bottom fixed to the base; a monitoring shield (30), pivotably connected to the top of the housing (20); a pair of open magnets (40), which are spaced apart from each other and fixed to the base (10) by a magnet holder (50) such that an imaging area is defined between them; an operating table (60), fixed in the imaging area; an incubator (70), movably connected to the operating table (60) and configured to house an infant or child and to adjust the position of the infant or child in the imaging area. The monitoring shield (30) has a closed configuration and an open configuration. In the closed configuration of the monitoring shield (30), the magnet holder (50), the open magnet (40), the operating table (60) and the incubator (70) are all situated within a space delimited by the base (10), the housing (20) and the monitoring shield (30). With this optimized structure, the system allows a radiologist to more accurately and intuitively adjust and understand the position and angle at which the infant or child is imaged. In addition, with the incubator (70), the system can provide the infant or child with a safer and more comfortable environment. Therefore, it entails a systematic MRI solution for newborns, infants and children.

## Description

### TECHNICAL FIELD

The present invention relates to a magnetic resonance imaging (MRI) system for infants and children and imaging method thereof.

### BACKGROUND

Magnetic resonance imaging (MRI) systems are often used in the field of medical and health care. Hydrogen atomic nuclei (protons) in body tissues can release nuclei magnetic resonance signals when excited by radio frequency pulses in a magnetic field. MRI is an imaging technique which allows computerized reconstruction of cross-sectional body image slices from the nuclei magnetic resonance signals.

In the medical field, more and more attention has been paid to the use of imaging diagnosis for common diseases in infants and children. Among the techniques most commonly used for this purpose, including MRI, CT and sonography, MRI is remarkably advantageous due to its merits in safety and imaging quality. Nowadays, there are a number of commercially available MRI systems specially designed for infants and children. However, they are limited in terms of openness and applicability. For example, in these systems, an infant or a child is fully accommodated within a dedicated enclosure, which impedes the radiologist's observation and creates a potential risk in safety. In addition, these conventional MRI systems have some applicability issues such as a high noise level, low controllability of exposure to electromagnetic radiation and absence of a sophisticated insulation measure.

### SUMMARY OF THE INVENTION

It is an object of the present invention to provide an MRI system for infants and children and imaging method thereof to solve the problems arising from conventionally performing an MRI process on an infant or a child such as absence of compatible insulation measures and incapability of the infant or child to change the body position according to the radiologist's instructions.

This object is attained by an MRI system for infants and children according to the present invention, which comprises: a base; a housing, with a bottom thereof fixed to the base; a monitoring shield, pivotably connected to the top of the housing; open magnets, which are spaced apart from each other and fixed to the base by a magnet holder such that an imaging area is defined between them; an operating table, fixed in the imaging area; and an incubator, movably connected to the operating table and configured to house an infant or a child and to adjust the position of the infant or child in the imaging area. The monitoring shield has a closed configuration in which the operating table and the incubator are electromagnetically shielded from an external environment and an open configuration in which the operating table and the incubator are exposed to the external environment. In the closed configuration of the monitoring shield, each of the magnet holder, the open magnets, the operating table and the incubator is situated within a space delimited by the base, the housing and the monitoring shield.

Preferably, the MRI system further comprises an amplifier for amplifying a nuclear magnetic resonance (NMR) signal generated in the imaging area and a spectrometer for analyzing the NMR signal.

Preferably, the incubator is provided with a radio frequency (RF) receiving coil.

Preferably, a gradient coil and a shim coil are arranged around the open magnets.

Preferably, the gradient coil is a quiet gradient coil.

Preferably, the monitoring shield is made of a transparent material.

The present invention also provides a MRI method for infants and children, which uses the MRI system as defined above and comprises the steps of:
S1: placing the infant or child in the incubator;
S2: closing the monitoring shield; and
S3: performing an MRI process after a temperature of the incubator as well as the position thereof relative to the open magnets have been so adjusted that a target site of the infant or child is situated within the imaging area.

Compared to the prior art, the system and method according to the present invention offer the following advantages:
1. With the optimized structure, particular open magnet arrangement and specially designed incubator and operating table, a radiologist can more accurately and intuitively adjust and understand the position and angle at which the infant or child is imaged.
2. Use of the quiet gradient coil can reduce the noise level and maintain a quiet and comfortable environment for the infant or child.
3. The transparent material of the monitoring shield allows the radiologist and parent(s) to more directly observe the whole diagnostic process being performed on the infant or child and hence provides a better user experience.
4. With the open magnet, the optimized RF coil design and the dedicated sequence design for infants and children, exposure to radiation energy can be reduced.

### BRIEF DESCRIPTION OF THE DRAWINGS

Fig.1 is a schematic, three-dimensional illustration of an MRI system for infants and children (with a monitoring shield thereof being in an open configuration) in accordance with a particular embodiment of the present invention.
Fig. 2 is a front view of Fig. 1.
Fig. 3 is a top view of Fig. 1.
Fig.4 is a schematic, three-dimensional illustration of an MRI system for infants and children (with a monitoring shield thereof being in a closed configuration) in accordance with a particular embodiment of the present invention.

In these figures, 10 denotes a base; 20, a housing; 30, a monitoring shield; 31, sealing edges; 40, an open magnet; 50, a magnet holder; 60, an operating table; 70, an incubator; and 80, an amplifier and a spectrometer.

### DETAILED DESCRIPTION

In order to more fully describe the subject matter of the present invention, several particular embodiments are listed below for demonstration of its technical effects. It is noted that these examples are provided for illustration only, without limiting the scope of the invention in any way.

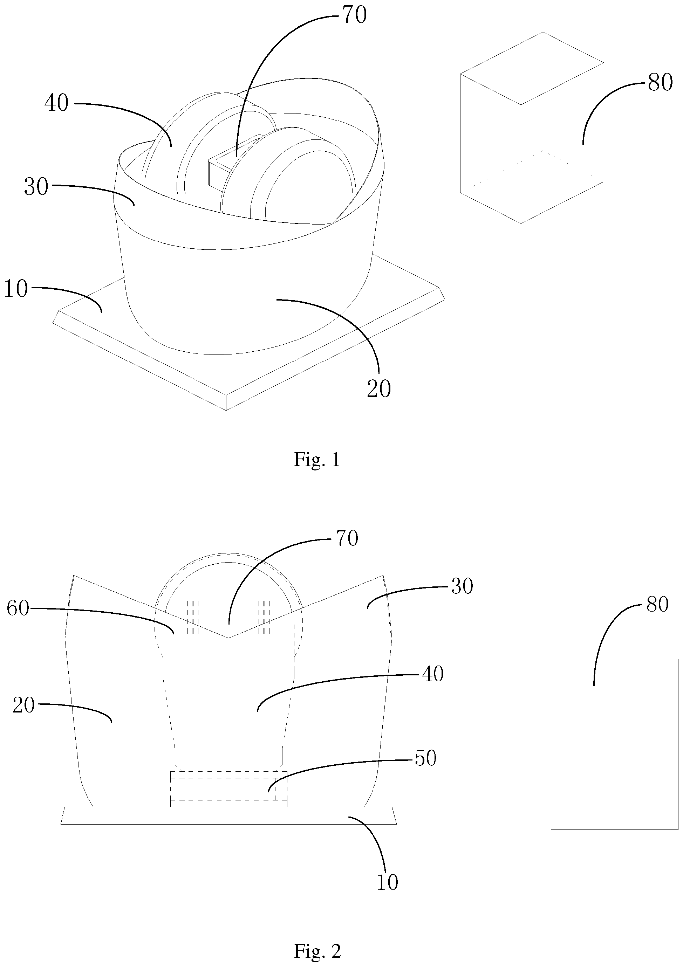

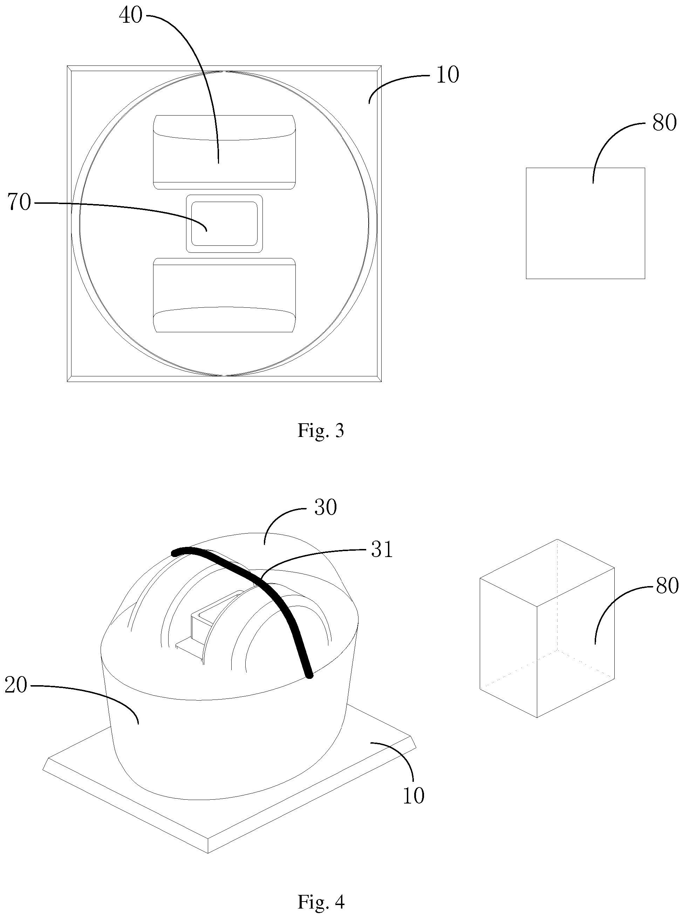

As shown in Figs. 1 to 4, the present invention provides a magnetic resonance imaging (MRI) system for infants and children, which includes a base 10, a housing 20, a monitoring shield 30, an open magnet 40, a magnet holder 50, an operating table 60 and an incubator 70. Of course, it may further comprise other necessary MRI components, which are not described herein for the sake of simplicity. Specifically, the housing 20 is fixed to the base 10 with a bottom and is pivotably connected at the top to the monitoring shield 30 so that the monitoring shield 30 can have an open configuration allowing an infant or child to be put in or taken out and a closed configuration in which an MRI process is carried out (referring to Figs. 1 and 4). An electromagnetic shielding layer (not shown) may be provided in the housing 20, and the magnet holder 50 may be provided with a transmission board (not shown) for wiring as well as for shielding of electromagnetic signals. The monitoring shield 30 is connected to the electromagnetic shielding layer in the housing 20 and the electromagnetic shielding layer is connected, in turn, to the transmission board, so that when the monitoring shield 30 is closed with its two halves in contact with each other, with sealing edges 31 formed at their middle, the monitoring shield 30, the electromagnetic shielding layer and the transmission board together delimit an electromagnetically shielded space. Therefore, the MRI system can provide a complete electromagnetic shielding which is beneficial for the infant or child. The magnet holder 50 is fixed to the base 10, and the open magnet 40 is fixed to the magnet holder 50. The open magnet 40 defines an imaging area in which the operating table 60 is fixedly disposed. In a specific embodiment, the upper portions of the open magnet 40 are consisted of two semicircular magnets which are spaced apart from each other, and the imaging area is located between them. The incubator 70 is movably connected to the operating table 60. That is, the incubator 70 is movable freely in the imaging area relative to the open magnet 40, for example, translating horizontally or vertically, or rotating about a vertical axis. In practice, the incubator can be designed to move in the same manner as any conventional system, and further description in this regard is omitted herein because movement of incubator is not the focus of the present invention. It is a matter of course that a temperature of the incubator 70 is adjustable. The magnet holder 50, the open magnet 40, the operating table 60 and the incubator 70 are all disposed within a space defined by the base 10, the housing 20 and the monitoring shield 30. With the optimized structure, particular arrangement of the open magnet 40 and specially designed incubator 70 and operating table 60, the MRI system according to the present invention allows a radiologist to more accurately and intuitively adjust and understand the position and angle at which the infant or child is imaged with the latter being always held in a warm and comfortable environment.

Preferably, with focused reference to Fig. 1, the MRI system may further comprise an amplifier and a spectrometer 80, and a radio frequency (RF) receiving coil 71 may be arranged in the incubator 70. A gradient coil and a shim coil 41 may be disposed around the open magnet 40 such that each of the open magnet 40, the gradient coil and the shim coil 41 is located within the housing. In a specific embodiment, nuclear magnetic resonance occurs under the cooperation of the open magnet 40, the gradient coil and the shim coil 41 around it, the RF receiving coil 71, the associated amplifier and spectrometer (spectrum analyzer) 80, and an imaging workstation. The cooperation is based on the principle of nuclear magnetic resonance in which protons in an object being imaged spin in a main magnetic field generated by the open magnet 40 where a spatial encoding is induced by the gradient coil, and resonance signals are excited and received by the RF coil. The resonance signals are further used by the imaging workstation for image reconstruction. In MRI applications, the patient's body portion or an object is positioned in the imaging area around the center of the open magnet 40. It is a matter of course that the imaging area may be determined by the design and placement of the components of the MRI system. With the infant or child being placed in the incubator 70 that is provided with the embedded RF receiving coil, a connection is established between the power supply, signal, air circulation and other interfaces in the incubator 70 and the dedicated operating table 60 which is, in turn, connected to the imaging workstation to accept control commands such that the body position of the infant or child can be adjusted by changing the relative position of the incubator 70 in order to accurately and easily locate a target site into the imaging area with the infant or child being maintained in a safe and comfortable environment.

Preferably, the gradient coil is a quiet gradient coil provided with a vacuum insulation layer which can minimize the noise level during the imaging process and hence provide the infant or child with a quiet and comfortable accommodation.

Preferably, the monitoring shield 30 is made of a transparent material which allows the radiologist and parent(s) to more directly observe the whole diagnostic process to make sure the infant or child is in a good condition and hence provides a better user experience while imparting a sufficient shielding ability to the monitoring shield 30.

In addition, with the open magnet 40, the optimized RF coil design and the dedicated sequence design for infants and children, the MRI system according to the present invention can reduce the exposure to radiation energy and is therefore more suitable for infant and child patients.

The present invention also provides an MRI method for infants and children, using the above-described MRI system and comprising the steps of:
S1: placing the infant or child in the incubator 70;
S2: closing the monitoring shield 30; and
S3: performing an MRI process after a temperature of the incubator 70 as well as the position thereof relative to the open magnet 40 have been so adjusted that a target site of the infant or child is situated within the imaging area.

In summary, the present invention provides an MRI system for infants and children and imaging method thereof. The system comprises a base 10, a housing 20, a monitoring shield 30, an open magnet 40, a magnet holder 50, an operating table 60 and an incubator 70. The housing 20 is secured at the bottom to the base 10 and is pivotably connected at the top to the monitoring shield 30. The monitoring shield 30, together with an electromagnetic shielding layer in the housing 20 and a transmission board, provides a complete electromagnetic shield for the MRI system. The magnet holder 50 is fixed to the base 10, and the open magnet 40 is fixed to the magnet holder 50. The open magnet 40 defines an imaging area in which the operating table 60 is fixedly disposed. The incubator 70 is movably connected to the operating table 60. The magnet holder 50, the open magnet 40, the operating table 60 and the incubator 70 are all disposed within a space delimited by the base 10, the housing 20 and the monitoring shield 30. With the optimized structure, particular arrangement of the open magnet 40 and specially designed incubator 70 and operating table 60, the MRI system according to the present invention allows a radiologist to more accurately and intuitively adjust and understand the position and angle at which the infant or child is imaged with the latter being always held in a warm and comfortable environment.

It will be apparent to those skilled in the art that various changes and modifications can be made to the invention without departing from the spirit and scope thereof. Accordingly, it is intended that the present invention also include these modifications and variations if they come within the scope of the appended claims and their equivalents.

## Claims

1. A magnetic resonance imaging (MRI) system for infants and children, comprising:
a base;
a housing, with a bottom thereof fixed to the base;
a monitoring shield, pivotably connected to a top of the housing;
open magnets, which are spaced apart from each other and fixed to the base by a magnet holder such that an imaging area is defined between the open magnets;
an operating table, fixed in the imaging area; and
an incubator, movably connected to the operating table and configured to house an infant or child and to adjust a position of the infant or child in the imaging area,
wherein the monitoring shield has a closed configuration in which the operating table and the incubator are electromagnetically shielded from an external environment and an open configuration in which the operating table and the incubator are exposed to the external environment, and wherein in the closed configuration of the monitoring shield, each of the magnet holder, the open magnets, the operating table and the incubator is situated within a space delimited by the base, the housing and the monitoring shield.

2. The MRI system for infants and children of claim 1, further comprising: an amplifier for amplifying a nuclear magnetic resonance (NMR) signal generated in the imaging area; and a spectrometer for analyzing the NMR signal.

3. The MRI system for infants and children of claim 1, wherein the incubator is provided with a radio frequency (RF) receiving coil.

4. The MRI system for infants and children of claim 1, wherein a gradient coil and a shim coil are arranged around the open magnets.

5. The MRI system for infants and children of claim 4, wherein the gradient coil is a quiet gradient coil.

6. The MRI system for infants and children of claim 1, wherein the monitoring shield is made of a transparent material.

7. A magnetic resonance imaging (MRI) method for infants and children, using an MRI system as defined in any one of claims 1 to 6 and comprising the steps of:
S 1: placing the infant or child in the incubator;
S2: closing the monitoring shield; and
S3: performing an MRI process after a temperature of the incubator as well as a position thereof relative to the open magnets have been so adjusted that a target site of the infant or child is situated within the imaging area.
